Europäisches Patentamt

European Patent Office

Office européen des brevets

(11) Veröffentlichungsnummer: **0 099 524**
**A2**

(12) # EUROPÄISCHE PATENTANMELDUNG

(21) Anmeldenummer: 83106693.1

(22) Anmeldetag: 08.07.83

(51) Int. Cl.³: **G 02 C 7/10**

(30) Priorität: 10.07.82 DE 3225863

(43) Veröffentlichungstag der Anmeldung:
01.02.84 Patentblatt 84/5

(84) Benannte Vertragsstaaten:
AT BE CH DE FR GB IT LI LU NL SE

(71) Anmelder: Kunert, Heinz, Dr.
Am Krieler Dom 23
D-5000 Köln 41(DE)

(72) Erfinder: Kunert, Heinz, Dr.
Am Krieler Dom 23
D-5000 Köln 41(DE)

(74) Vertreter: Geitz, Heinrich, Dr.-Ing.
Postfach 2708 Kaiserstrasse 156
D-7500 Karlsruhe 1(DE)

(54) Augenvorsatzscheibe, insbesondere für Kraftfahrzeuglenker.

(57) Die Augenvorsatzscheibe, bei der es sich um Brillengläser, Haftschalen, aber auch Visiere für Schutzhelme handeln
kann, besitzt ein sich quer über die Scheibe erstreckendes, zu
den seitlichen Scheibenrändern hin verbreiterndes Hauptsichtfeld, an das sich unter- und oberhalb Felder anschließen, deren Transmission gegenüber der Transmission des
Hauptsichtfeldes reduziert ist, so daß diese Felder einen
wirksamen Blendschutz für von unten und oben einfallende
Lichtstrahlen vermitteln. Die Felder mit verringerter Transmission können bereits in Zonen unterteilt sein, wobei die
Transmission der vom Hauptsichtfeld entfernten Zonen
stärker reduziert ist als die der am Hauptsichtfeld anliegenden Zonen. Die Begrenzungslinien zwischen dem Hauptsichtfeld und den an diesem anliegenden Zonen verringerter
Transmission einerseits und zwischen diesen Zonen und
dem von Hauptsichtfeld entfernten Zonen verlaufen in der
Weise gekrümmt, daß sich das Hauptsichtfeld zu den
seitlichen Scheinbenrändern hin verbreitert und dadurch
eine Erweiterung des Blickfeldes im temporalen Bereich, und
bei der Ausgestaltung als Brillengläser, auch im nasalen
Bereich eintritt.

FIG. 1

DR. ING. HEINRICH GEITz PATENTANWALT 7500 KARLSRUHE 1, POSTFACH 2708

832096

Anmelder:  Dr. Heinz Kunert
           Am Krieler Dom 23
           D-5000 Köln 41

Augenvorsatzscheibe, insbesondere für Kraftfahrzeug-
                      lenker
=======================================================

Die Erfindung bezieht sich auf eine Augenvorsatzscheibe mit einem sich im wesentlichen horizontal
erstreckenden Hauptsichtfeld und unter und über
letzterem angeordneten Feldern mit im Vergleich zum
Hauptsichtfeld verringerter Transmission im sichtbaren Bereich.

Die erfindungsgemäße Augenvorsatzscheibe soll insbesondere für Kraftfahrzeuglenker geeignet und bestimmt, nicht aber auf diesen Anwendungsfall beschränkt sein. Unter Augenvorsatzscheiben im Sinne
der vorliegenden Erfindung sind beispielsweise Brillengläser und Kontaktlinsen, aber auch transparente
Visiere bei Schutzhelmen zu verstehen.

Der obere Bereich der Netzhaut des menschlichen Auges, auf dem vom Auge erfaßte Gegenstände am Boden oder im Nahbereich abgebildet werden, besitzt eine erhöhte Sensibilität. Dies hat einen phylogenetischen Bezug. Für die Hominiden und vorgeschichtlichen menschlichen Vorfahren war eine gute Orientierung bei Nacht und Dunkelheit über das Sehen im nahen Fußraum von lebenswichtiger Bedeutung. Sie mußten auf dem Boden liegende gefährdende Hindernisse und Gefahrenquellen sicher erkennen. Insofern bildete die Ausprägung dieser spezifischen Sensibilität einen Selektionsvorteil.

Gegenüber einfallenden Lichtstrahlen von Lichtquellen oberhalb der horizontalen Blickebene ist dieser obere Netzhautbereich des Auges durch die Augenwülste und Augenbrauen geschützt. Auch diese Körpermerkmale haben sich in Anpassung an die Umwelt herausgebildet. In der damaligen Umwelt war der Mensch nur der Sonne als einer oberhalb der Blickebene stahlenden Licht- und Blendungsquelle ausgesetzt. Für die Ausbildung von entsprechenden Wülsten unterhalb der Augen als Schutz vor unterhalb der Sichtebene einstrahlenden Licht- oder Blendungsquellen bestand hingegen keine Notwendigkeit, weil es für den frühen Menschen als Wald- und Steppenbewohner keine vom Boden reflektierten blendenden Sonnenstrahlen gab. Die Wiege der Menschheit stand nicht in kalten, schneebedeckten Zonen.

Auch der heutige Mensch ist gegen den direkten Einfall des Sonnenlichtes durch seine Augenwülste und Augenbrauen geschützt, nicht aber gegen Reflextionen der Sonneneinstrahlung, die beispielsweise an der Oberfläche von Fahrbahnen, Motorhauben von Kraftfahrzeugen und ähnlichen Reflextionsflächen auftreten. Eine derartige Reflexionsstrahlung kann durchaus die gleiche Intensität wie direkte Sonneneinstrahlung haben. Desgleichen ist der Mensch ohne Schutz gegenüber bei Dunkelheit und Nacht von Blendquellen unterhalb der Sichtebene einfallenden Lichtstrahlen. So ist der Lenker eines Kraftfahrzeugs den von der Fahrbahn reflektierten Lichtstrahlen seiner eigenen Scheinwerfer ungeschützt ausgesetzt.

Ausgehend von dieser Erkenntnis besteht das Ziel der vorliegenden Erfindung in der Schaffung einer Augenvorsatzscheibe, die einen wirksamen Schutz des menschlichen Auges gegenüber Blendqeu-llen auch unterhalb der Blickebene vermitteln und insbesondere auch ein weitgehend blendfreies Nacht- und Dämmerungssehen ermöglichen.

Eine für diesen Zweck bestimmte Brille ist bereits in der US-PS 24 09 356 vorbeschrieben, deren Gläser ein sich horizontal erstreckendes Hauptsichtfeld in Form eines quer über die Gläser verlaufenden Bandes gleichbleibender Breite und unter- und oberhalb vom Hauptsichtfeld angeordnete Felder mit gegenüber dem Hauptsichtfeld erhöhten Absorptionsvermögen aufweisen. Mit so gestalteten Brillengläsern wird zwar, sofern die Transmission der unter- und oberseitig an das Hauptsichtfeld anschließenden Felder entsprechend bemessen ist, ein guter Blendschutz auch gegenüber von unten einfallenden Lichtstrahlen erreicht, gleichzeitig aber auch eine das Nahsehvermögen stark behindernde Einengung des Blickfeldes im nasalen und temporalen Bereich.

Durch die vorliegende Erfindung soll diesen Unzulänglichkeiten abgeholfen und eine Augenvorsatzscheibe geschaffen werden, die in dem für die Wahrnehmung von Bewegungsvorgängen wichtigen peripheren Bereich das Blickfeld nicht einengt und einen wirksamen Blendschutz auch für das dunkel adaptierte Auge vermittelt.

Diese Aufgabe ist bei einer Augenvorsatzscheibe nach dem Oberbegriff des Patentanspruchs 1, wie sie eingangs beschrieben ist, dadurch gelöst, daß sich das Hauptsichtfeld hinsichtlich seiner vertikalen Erstreckung von seinem mittleren Teil zu den peripheren Bereichen hin verbreitert.

Im Gegensatz zu dem als sich quer über die Brillengläser erstreckendes Band gleicher Breite, bei den ausgebildeten Hauptsichtfeld Gläsern der vorbekannten Brille nach der US-PS 24 09 356 treten bei der erfindungsgemäßen Augenvorsatzscheibe die im Vergleich zum Hauptsichtfeld verringerte Transmission aufweisenden Felder, die sich unter- und oberhalb an das Hauptsichtfeld anschließen, mit größer werdendem Abstand von dem mittleren Teil des Hauptsichtfeldes zurück, so daß angesichts der dadurch sich nach beiden Seiten der Scheibe hin ergebenden Verbreiterung des Hauptsichtfeldes in den seitlichen Scheibenbereichen eine Vergrößerung des Blickfeldes und damit eine Verbesserung der Wahrnehmungsmöglichkeiten insbesondere in den genannten peripheren Bereichen eintritt.

Eingehende Versuche haben gezeigt, daß Kraftfahrer,
die mit erfindungsgemäß ausgebildeten Augengläsern
ausgerüstet waren, selbst bei Fahrten mit Abblendlicht ohne Blendung durch entgegenkommende Fahrzeuge
einen Zuwachs an Sichtdistanz von durchschnittlich
7% hatten. Beim Tragen derartiger Brillengläser
konnten im Vergleich zu durchgängig transparenten
Brillengläsern auch Vergrößerungen der Pupillenweite geme-ssen werden. Dies bestätigt die direkt
gewonnenen Untersuchungsergebnisse über den Zuwachs
an Sichtdistanz. Die Vergrößerung der Pupillenöffnung ist darüber hinaus ein dirkter Indikator
für die erzielte Blendreduktion und für den Gewinn
an Information bei Dunkelheit und Nacht.

Nachdem vorliegende Untersuchungen gezeigt haben,
daß sich der Kraftfahrer insbesondere bei Fahrten
in der Dunkelheit und bei Nacht über die unterschwellige Wahrnehmung des seitlichen Konturenverlaufs der Straße oder der Fahrbahn mit Hilfe der
Abbildung auf peripheren Teilen der Netzhaut
orientiert, eignet sich die erfindungsgemäße Ausbildung der Augenvorsatzscheibe insbesondere für
die Fahrer von Kraftfahrzeugen. Vornehmlich bei
dieser Verwendung spielt nicht so sehr die Konturierung im Fernraum, sondern im unmittelbaren
Nahraum eine wesentliche Rolle. Erstere werden
mehr bewußt und über das zentrale Netzhautfeld
wahrgenommen, während die peripheren Netzhautfelder eine ausgesprochen hohe Sensibilität für das Sehen von bewegten oder plötzlich in das Gesichtsfeld eintretenden Objekten aufweisen (Leit- und Warnfunktion des peripheren Sehens). Die Bedeutung dieses Phänomens erkennt man bei Fahrten auf Straßen mit fehlenden oder unkonturierten Begrenzungen. Erst die deutlichen und scharf konturierten Leitlinien und Fahrbahnbegrenzungen, etwa mittels weißer Farbmarkierungen, ermöglichen ein sicheres, wenig ermüdendes und zügiges Fahren bei Dunkelheit und Nacht, desgleichen bei Zwielicht und Blendung. Die erschwerten Fahrbedingungen bei Dunst und Nebel verdeutlichen die hier gegebenen physiologischen Gesetzmäßigkeiten gleichfalls.

Diesen Erfordernissen genügt die erfindungsgemäße Augenvorsatzscheibe in hervorragender Weise insofern, als sich das Hauptsichtfeld, wie vorbekannt, horizontal über einen Winkelbereich von ca. 180° erstreckt und, wie bereits ausgeführt, in den peripheren Bereichen breiter gestaltet ist als im Mittelfeld, so daß dem Bedürfnis für eine sichere Orientierung im bewegten Zustand auf die Wahrnehmung von Objekten im unmittelbaren peripheren Nahraum in einer möglichst großen Breite vor allem unter- und oberhalb der horizontalen Sichtebene genügt ist.

Vorteilhafte Ausgestaltungen der Erfindung sind in den Unteransprüchen angegeben.

So besteht eine zweckmäßige Weiterbildung der Augenvorsatzscheibe darin, daß die unter- und oberhalb des Hauptsichtfeldes positionierten Felder mit im

Vergleich zum Hauptsichtfeld verringerter Transmission gegenüber dem Hauptsichtfeld gekrümmte Begrenzungen aufweisen. Dabei kann wenigstens das unterhalb des Hauptsichtfeldes angeordnete Feld verringerter Transmission aus zwei im wesentlichen horizontal orientierten gekrümmten Zonen bestehen, wobei die an das Hauptsichtfeld angrenzende Zone eine höhere Transparenz aufweist als die vom Hauptsichtfeld entfernte Zone.

Eine andere vorteilhafte Ausgestaltung der Erfindung besteht darin, daß die an das Hauptsichtfeld angrenzenden Zonen einen zum Hauptsichtfeld hin verlaufenden Gradienten mit zunehmender Transmission aufweisen.

Ferner hat es sich als zweckmäßig erwiesen, wenn das Hauptsichtfeld hohe Transmissionswerte von wenigstens 85% besitzt, also nahezu ganz durchlässig ist oder nur schwaches Absorptionsvermögen aufweist. Die an das Hauptsichtfeld angrenzenden Felder sollten demgegenüber eine Transmission von höchstens 70% haben. Bei der Aufteilung der Felder mit im Vergleich zum Hauptsichtfeld verringerter Transmission in mehrere Zonen oder mit einem zum Hauptsichtfeld hin verlaufenden Gradienten mit zunehmender Transmission sollten die vom Hauptsichtfeld entfernten Felder eine Transmission von höchstens 30% aufweisen.

Eine weitere zweckmäßige Ausgestaltung ist dadurch gekennzeichnet, daß das Hauptsichtfeld symmetrisch zu einer die Gesichtslinie schneidenden Horizontalen
angeordnet und nach unten und oben in den Scheitelpunkten der angrenzenden gekrümmten Begrenzungen
durch einen Blickwinkel von je etwa 4° begrenzt
ist. Bei der Aufteilung der Felder mit im Vergleich
zum Hauptsichtfeld geringerer Transmission in mehrere
Zonen sollten die Scheitelpunkte der gekrümmten
Zonen mit geringster Transparenz von einer die Gesichtslinie schneidenden Horizontalen je einen Abstand haben, der einem Blickwinkel von etwa 10°
entspricht.

Eine andere zweckmäßige Ausgestaltung besteht darin,
daß der Übergang von den Zonen mit der geringsten
Transmission zu den Zonen mit nur geringerer Transmission graduell verläuft. Auch kann der Übergang
von den Zonen mit geringerer Transmission zum Hauptsichtfeld graduell verlaufen und zweckmäßigerweise
durch die im Patentanspruch 11 bzw. die im Patentanspruch 12 angegebenen  Funktion bestimmt sein.

Zum Verringern der Transparenz bestimmter Feldbereiche der erfindungsgemäßen Augenvorsatzscheiben
kann beispielsweise ein Graufilter dienen, der mit
transparenten nicht streuenden Farben ausgebildet
wird, vorzugsweise als Farbmischung von blau/grün,
gelb und purpur der Lichtechtheitsklassen VI bis VIII.
Die Filterwirkung kann aber auch durch Einfärbung
der transparenten Felder etwa über die gesamte Scheibendicke oder auch durch Einfärbung einer unter der
Außenschicht der Scheibe liegenden Schicht erreicht werden. Eine andere Möglichkeit, die Filterwirkung zu erreichen, besteht in der Einfärbung einer Außenschicht der Scheibe sowie darin, daß diese Außenschicht einen Brechungsindex aufweist, der dem der transparenten Scheibe entspricht.

Für die Gestaltung von Augenvorsatzscheiben, die geeignet sind, sichtreduzierende Effekte, insbesondere solche durch Blendung, zu vermeiden, ist aber auch noch das Phänomen des Lichtleitereffekts in transparenten Platten beachtlich.

Augenvorsatzscheiben haben zumeist eine nach außen gewölbte Form. Die äußere Oberfläche ist in der Regel konvex ausgebildet. Beim Ablegen solcher Scheiben, wie Brillen, Helmvisiere und dgl., wird der mittlere Teil der äußeren Oberfläche bevorzugt durch Verkratzungen beschädigt. Die Innenflächen von Augengläsern sind meist konkav geformt. Beim Putzen solcher Gläser erfährt daher bevorzugt der mittlere Teil der Innenfläche durch die mechanische Manipulation Aufrauhungen und Verkratzungen.

Unter einem Grenzwinkel in eine transparente Platte eintretende Lichtstrahlen werden in der Platte total reflektiert. Diese Totalreflektion an den inneren Grenzflächen eines transparenten Körpers aus Glas oder Kunststoff zu der ihn umgebenden Luft führt zu Mehrfach-Reflektion, also zu einer Lichtleitung, wie sie beispielsweise bei Glasstäben oder Glasfasern systematisch genutzt wird.

Bei in einem flachen Winkel auffallenden Lichtstrahlen tritt stets ein Teil dieser Strahlung
unter den eine Totalreflektion auslösenden Bedingungen in einen transparenten Körper ein.
Doch auch senkrecht, oder in einem stumpfen Winkel
auffallende Lichtstrahlen können, sofern sie auf
Streuzentren auftreffen, die durch Oberflächen-beschädigungen, Kondensfilme oder Staubkörnchen gebildet sein können, in einer für eine Totalreflektion
notwendigen Winkelgröße in die transparente Platte
eintreten.

Solche über der gesamten Fläche von Augenvorsatzscheiben unter den genannten Bedingungen eintretende Lichstrahlen pflanzen sich über Mehrfach-Reflektion
über das Innere des Scheibenkörpers flächenhaft
fort. Sie treten andererseits an den genannten
Streuzentren oder Streupartikelchen wieder aus
dem Glaskörper aus.

Wenn im mittleren Bereich solcher Augengläser nunmehr, wie beschrieben, nach längerem Gebrauch sich
"streuende" Oberflächen bilden, etwa infolge Verkratzungen, so treten hier in größerer Dichte Lichtstrahlen, die in Randzonen eingetreten sind, wieder
aus.Sie fallen dann zum größten Teil direkt auf
zentrale Netzhautbereiche.

Das Auge nimmt derartige Flächen als schwach selbstleuchtend wahr. Die so entstehende spezifische Leuchtdichte kann als Leuchtdichte über der Fläche gemessen
werden. In dem diese Schleierleuchtdichte die Leuchtdichte des durch die transparente Platte zu beobachtenden Objektes bzw. dessen Umfeld-Leuchtdichte überlagert, benötigen Objekte größere Kontrastschwellen um wahrgenommen zu werden. Dieses Phänomen wird als Schleier-Leuchtblendung bezeichnet. Deren Störeffekt hängt im wesentlichen von der Intensität der Schleierleuchtdichte und der Flächengröße ab.

Hinsichtlidh dieses Phänomens schafft eine weiterbildende Ausgestaltung der Erfindung dadurch Abhilfe, daß zur Unterbrechung der Lichtleitung in der Augenvorsatzscheibe von den peripheren Feldern zum zentralen Hauptsichtfeld in der Scheibe eine parallel zu den Scheibenoberflächen verlaufende Schicht mit lichtabsorbierenden Eigenschaften vorgesehen ist, beispielsweise eine eingefärbte Schicht, die je nach ihrer Einfärbung die Lichtleitung über Mehrfach-Reflexion über kurze Wege reduziert. Auf diese Weise kann die Lichtleitung in der Scheibe unterbrochen oder, je nach Bedarfsfall, auch nur reduziert werden.

Eine andere zweckmäßige Maßnahme, die Lichtleitung in der Augenvorsatzscheibe zu verhindern oder abzuschwächen, besteht darin, daß sich wenigstens außerhalb des Hauptsichtfeldes im Abstand voneinander verlaufende Farbbänder ohne oder zumindest geringer Transparenz in der Scheibe erstrecken, die im wesentlichen über die gesamte Dicke der Scheibe reichen. Dadurch ist die Lichtleitung in der als transparente Platte anzusehenden Augenvorsatzscheibe von den peripheren Feldern zum zentralen Hauptsichtfeld unterbunden oder zumindest stark reduziert.

Zweckmäßige Weiterbildungen dieser Maßnahme bestehen darin, daß die Farbbänder ohne oder zumindest geringer Transparenz unter einem von der Senkrechten zur Scheibenoberfläche abweichenden Winkel angeordnet sind, und zwar nach der Sichtstrahlen-Geometrie des auf den Fernpunkt gerichteten Auges. Besonders vorteilhaft ist es, wenn die Farbbänder ohne oder zumindest geringer Transparenz sich längs der Brenzungen zwischen den Feldern unterschiedlicher Transmission erstrecken. Um den Eintritt von Streulicht vom Rand der Augenvorsatzscheibe auszuschließen, ist zweckmäßigerweise die Außenkante der Scheibe von einer im wesentlichen über die Scheibendicke reichenden Schicht umschlossen, die gleichfalls keine oder zumindest nur geringe Transparenz aufweist.

Anhand der beigefügten Zeichnung sollen einige Ausführungsbeispiele der Erfindung erläutert werden. In schematischen Ansichten zeigen:

Fig. 1    eine als Brillenglas ausgebildete Augenvorsatzscheibe mit einem sich quer über die gesamte Breite erstreckenden und von seinem mittleren Teil nach beiden Seiten verbreiterten Hauptsichtfeld, das unter- und oberseitig von zwei jeweils in zwei Zonen unterteilten Feldern mit im Vergleich zur Transmission des Hauptsichtfeldes verringer Transmission eingeschlossen ist,

Fig. 2    eine erfindungsgemäß ausgebildete Skibrille,

Fig. 3      eine Augenvorsatzscheibe in Form eines
            transparenten Visiers zur Verwendung bei
            Schutzhelmen für Kraftradfahrer und

Fig. 4      verschiedene Möglichkeiten zur Reduktion
            des Transmissionswertes in den einzelnen
            Feldern der Augenvorsatzscheibe (Fig. 4a
            und 4b) bzw. zur Unterbindung oder Reduzierung des Lichtleitereffektes (Fig. 4c).

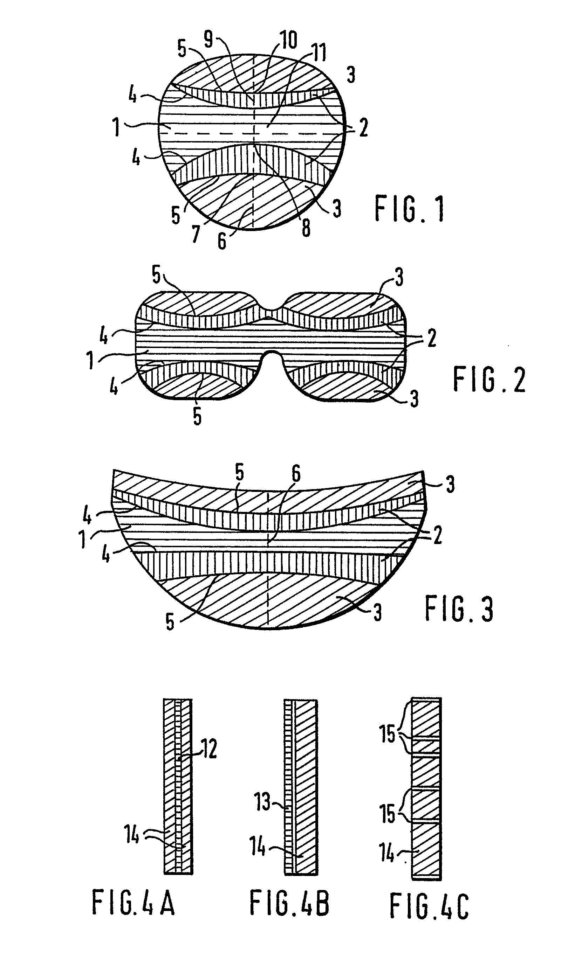

Bei dem in Fig. 1 veranschaulichten Brillenglas erstreckt sich das Hauptsichtfeld 1 etwa in der Mitte des Glases über dessen gesamte Breite und verbreitert sich mit zunehmendem Abstand von einer gedachten Mittelsenkrechten, die als gestrichelte Linie angedeutet und mit 6 bezeichnet ist, allmählich fortschreitend nach beiden Seiten. Ober- und unterseitig liegen an dem Hauptsichtfeld 1 von letzterem durch bogenförmige Begrenzungen 4 abgetrennte mondsichelartige Zonen 2 mit gegenüber der Transmission des Hauptsichtfeldes 1 verringerter Transmission an. Die Transmission der Zonen 2 beträgt höchstens 70%. Außenseitig, also auf den vom Hauptsichtfeld 1 entfernten Seiten, schließen sich an die Zonen 2 über ebenfalls bogenförmige Begrenzungen 5 Zonen 3 nochmals reduzierter Transmission von höchstens 30% an. Die genannten Zonen 2, 3 sind in Form von Farbkeilen ausgebildet und gehen quasi stufenlos ineinander über, d.h. die äußeren Zonen 3 in die Zonen 2 und die Zonen 2 in das Hauptsichtfeld 1.

Die Mittelsenkrechte 6 schneidet den optischen Mittelpunkt 11 des Brillenglases. Die Scheitelpunkte 7 bis 10 der Zonenbegrenzungslinien 4, 5 liegen auf der genannten Mittelsenkrechten. Die Begrenzunglinien 4 zwischen dem Hauptsichtfeld und den an letzterem anliegenden Zonen 2 haben einen kleineren Radius als die Begrenzungslinien 5 zwischen den Zonen 2 und 3. Die Breite des Hauptsichtfeldes im peripheren Bereich ist angesichts der vorstehend erwähnten Krümmungen der Zonenbegrenzungen 4 zwischen dem Hauptsichtfeld und den an diesem anliegenden Zonen 2 wesentlich größer als in dem von der Mittelsenkrechten 6 durchschnittenen mittleren Teil.

Im Falle von Brillengläsern für eine Kraftfahrzeug-Fahrerbrille wird der Scheitelpunkt 7 durch einen Lichtstrahl festgelegt, der in einer Entfernung von 5 bis 10 Metern vor einem Kraftfahrzeug von der Fahrbahn reflektiert in das Auge einfällt, dessen Fixierlinie die ebene Fahrbahn in etwa 50 Metern Entfernung schneidet. Für den Scheitelpunkt 8 gilt dies unter den gleichen Bedingungen für einen reflektierenden Lichtstrahl in 15 bis 20 Metern Entfernung von einem Kraftfahrzeug. Der Scheitelpunkt 9 hat von dem optischen Mittelpunkt 11 etwa die doppelte Entfernung wie der Scheitelpunkt 8, während die Scheitelpunkte 10 und 7 etwa gleiche Abstände zum optischen Mittelpunkt 11 haben.

Bei der in Fig. 2 veranschaulichten Skibrille sind für gleiche Gegenstände wie in Fig. 1 die gleichen Bezugszeichen verwendet. Das Hauptsichtfeld 1 erstreckt sich wiederum über die gesamte Breite der beiden nebeneinander angeordneten Brillengläser und besitzt jeweils im peripheren und nasalen Bereich wesentlich größere Breite als in der Mitte der Breitenerstreckung der beiden Gläser, also in den Bereichen der nicht weiter bezeichneten optischen Mittelpunkte der Gläser. In Abweichung von der oben beschriebenen Ausführungsform können bei der Anwendung auf eine Skibrille für das Hauptsichtfeld 1 und die sich unter- und oberhalb von diesem anschließenden Zonen 2, 3, die gegenüber dem Hauptsichtfeld bzw. untereinander durch gekrümmte Begrenzungslinien 4, 5 abgegrenzt sind, abweichende Transparenzwerte realisiert werden, etwa in der Weise, daß das Hauptsichtfeld 1 bereits einen Transmissionswert von nur 70% besitzt, hingegen die Zone 2 einen Transmissionswert von 40% und die Zone 3 einen Transmissionswert von etwa 10%. Auch bei dieser Ausführungsform ist, wie bei dem Brillenglas nach Fig. 1, das Hauptsichtfeld im peripheren Bereich und im nasalen Bereich breiter gestaltet als im Mittelfeld.

Die als Visier zur Verwendung bei Schutzhelmen für Kraftradfahrer ausgebildete Augenvorsatzscheibe nach Fig. 3 ist in bezug auf die gestrichelt angedeutete Mittelsenkrechte 6, die den optischen Mittelpunkt schneidet, symmetrisch ausgebildet. Auch bei dieser Ausführungsform sind für gleiche Teile wie in den Fig. 1 und 2 die gleichen Bezugszeichen verwendet. Das Hauptsichtfeld besitzt wiederum gegenüber seinem Mittelteil merkliche Verbreiterungen in seinen peripheren Bereichen und wird unterhalb und oberhalb durch Zonen 2 mit im Vergleich zum Hauptsichtfeld verminderter Transmission begrenzt. Die Begrenzungslinien 4 zwischen den Zonen 2 und dem Hauptsichtfeld 1 sind wiederum in einer die Verbreiterung des Hauptsichtfeldes zu dessen peripheren Bereichen hin gewährleistenden Weise gekrümmt. In ähnlicher Form wie bei dem Brillenglas nach Fig. 1 schließen sich an die Zonen 2 über gleichfalls gekrümmte Begrenzungen 5 Zonen 3 mit weiter reduzierter Transmission an.

Die Reduktion der Transmissionswerte in den Zonen 2 und 3 außerhalb des Hauptsichtfeldes 1 kann durch Kerneinfärbung des Scheibenkörpers 14 erfolgen.

Wie Fig. 4a veranschaulicht, kann auch als Träger der Einfärbung eine Folie 12 Verwendung finden, die zwischen zwei Schichten des Scheibenkörpers 14 eingebracht ist und diese Schichten des Scheibenkörpers miteinander verbindet.

Wenn die Transmissionsminderung durch Aufbringung dünner Schichten aus Metallen oder dielektrischen Substanzen realisiert wird, wie dies häufig bei Sonnenschutzbrillen geschieht, ist eine zusätzliche Beschichtung 13 notwendig (Fig. 4b), die entweder aus einer Kunststoffolie oder aus einer aufgedampften transparenten Schicht besteht. Die Schichten 12 und 13 sollten zweckmäßigerweise den gleichen Brechungsindex aufweisen wie der Scheibenkörper 14.

Bei der in Fig. 4c veranschaulichten Ausführungsform sind den Begrenzungslinien 4, 5 zwischen dem
Hauptsichtfeld 1 und den Zonen 2 einerseits und
letzteren und den Zonen 3 andererseits folgend
winklig zur Oberfläche des Scheibenkörpers lichtundurchlässige Bänder eingezogen, die über die Dicke
des gesamten Scheibenkörpers reichen. Die Winkel dieser
Bänder zur Oberfläche des Scheibenkörpers sind durch
die Sehstrahlen der jeweiligen Orte der Brenzungslinien definiert. Desgleichen ist die Außenkante
des Scheibenkörpers mit einer ebenfalls lichtundurchlässigen Schicht 15 umlaufend bedeckt. Durch
diese Maßnahmen ist der Lichtleitereffekt bei streuenden Gläsern auf das Hauptsichtfeld 1 unterbunden.

DR. ING. HEINRICH GEITZ PATENTANWALT 7500 KARLSRUHE 1, POSTFACH 2708

832096

Anmelder:    Dr. Heinz Kunert

             Am Krieler Dom 23

             D-5000 Köln 41

Patentansprüche
================

1.   Augenvorsatzscheibe mit einem sich im wesentlichen horizontal erstreckenden Hauptsichtfeld und unter und über letzterem angeordneten Feldern mit im Vergleich zum Hauptsichtfeld verringerter Transmission im sichtbaren Bereich, insbesondere Augenvorsatzscheibe für Kraftfahrzeuglenker, dadurch gekennzeichnet, daß sich das Hauptsichtfeld (1) hinsichtlich seiner vertikalen Erstreckung von seinem mittleren Teil zu den peripheren Bereichen hin verbreitert.

2.   Augenvorsatzscheibe nach Anspruch 1, dadurch gekennzeichnet, daß die unterhalb und oberhalb des Hauptsichtfeldes (1) positionierten Felder (2, 3) verringerter Transmission gegenüber dem Hauptsichtfeld gekrümmte Begrenzungen (4, 5) aufweisen.

3.    Augenvorsatzscheibe nach Anspruch 1 oder 2, dadurch gekennzeichnet, daß wenigstens das unterhalb des Hauptsichtfeldes (1) angeordnete Feld aus zwei im wesentlichen horizontal orientierten gekrümmten Zonen (2, 3) besteht, wobei die an das Hauptsichtfeld angrenzende Zone (2) eine höhere Transparenz aufweist, als die vom Hauptsichtfeld entfernte Zone (3).

4.    Augenvorsatzscheibe nach einem der Ansprüche 1 bis 3, dadurch gekennzeichnet, daß die an das Hauptsichtfeld (1) angrenzenden Zonen (2) einen zum Hauptsichtfeld hin verlaufenden Gradienten mit zunehmender Transmission aufweisen.

5.    Augenvorsatzscheibe nach einem der Ansprüche 1 bis 4, dadurch gekennzeichnet, daß das Hauptsichtfeld (1) hohe Transmissionswerte von mindestens 85% aufweist.

6.    Augenvorsatzscheibe nach einem der Ansprüche 1 bis 5, dadurch gekennzeichnet, daß die an das Hauptsichtfeld (1) angrenzenden Felder (2) eine Transmission von höchstens 70% aufweisen.

7.    Augenvorsatzscheibe nach einem der Ansprüche 1 bis 6, dadurch gekennzeichnet, daß die vom Hauptsichtfeld (1) entfernten Felder (3) eine Transmission von höchstens 30% aufweisen.

8.    Augenvorsatzscheibe nach einem der Ansprüche 1
bis 7, dadurch gekennzeichnet, daß das Hauptsichtfeld (1) symmetrisch zu einer die Gesichtslinie
schneidenden Horizontalen angeordnet und nach unten
und oben in den Scheitelpunkten der angrenzenden
gekrümmten Begrenzungen (4) durch einen Blickwinkel von je etwa 4° begrenzt ist.

9.    Augenvorsatzscheibe nach einem der Ansprüche
1 bis 8, dadurch gekennzeichnet, daß die Scheitelpunkte der gekrümmten Zonen (3) mit geringster
Transparenz von einer die Gesichtslinie schneidenden Horizontalen je einen Abstand haben, der einem
Blickwinkel von etwa 10° entspricht.

10.    Augenvorsatzscheibe nach einem der Ansprüche
1 bis 9, dadurch gekennzeichnet, daß der Übergang
von den Zonen (3) mit der geringsten Transmission
zu den Zonen (2) mit nur geringerer Transmission
graduell verläuft.

11.    Augenvorsatzscheibe nach einem der Ansprüche
1 bis 10, dadurch gekennzeichnet, daß der Übergang
von Zonen (2) mit geringerer Transmission zum Hauptsichtfeld (1) graduell verläuft und durch Funktion

$$F = \cos.^2 X$$

bestimmt ist, wobei X = r, abgeleitet aus dem Einheitskreis, sich auf die jeweiligen Höhen des Zonenfeldes
bzw. innerhalb des Zonenfeldes bezieht.

832096

12.   Augenvorsatzscheibe nach einem der Ansprüche 1
bis 10, dadurch gekennzeichnet, daß der Übergang von
Zonen (2) mit geringerer Transmission zum Hauptsichtfeld (1) graduell verläuft und durch die Funktion

$$\Delta E = \sqrt{(\Delta L)^2 + (\Delta A)^2 + (\Delta B)^2}$$

mit      E = (Empfindlichkeitseinheiten)

bestimmt ist, wobei für L,  A und B die Gleichungen

$$L = 116\, y - 16$$
$$A = 500\,(x-y)$$
$$B = 200\,(y-z)$$

gelten und x, y gemäß DIN 5033 zu ermittelnde Farbkoordinaten sind.

13.    Augenvorsatzscheibe nach einem der Ansprüche 1 bis 11, dadurch gekennzeichnet, daß zum Verringern der Transparenz ein Graufilter dient, der mit transparenten, nicht streuenden Farben ausgebildet wird, vorzugsweise als Farbmischung von blau/grün, gelb und purpur der Lichtechtheitsklassen VI-VIII.

14.    Augenvorsatzscheibe nach einem der Ansprüche 1 bis 12, dadurch gekennzeichnet, daß die Filterwirkung durch Einfärbung der transparenten Felder (2, 3) über die gesamte Scheibendicke erreicht wird.

15.    Augenvorsatzscheibe nach einem der Ansprüche 1 bis 13, dadurch gekennzeichnet, daß die Filterwirkung durch Einfärbung einer unter der Außenschicht der Scheibe liegenden Schicht (12, 13) erreicht wird.

16.    Augenvorsatzscheibe nach einem der Ansprüche 1 bis 14, dadurch gekennzeichnet, daß die Filterwirkung durch Einfärbung einer Außenschicht der Scheibe erreicht wird und daß diese Schicht einen Berechnungsindex aufweist, der dem der transparenten Scheibe entspricht.

17.    Augenvorsatzscheibe nach einem der Ansprüche 1 bis 15, dadurch gekennzeichnet, daß in Parallellage zu den Scheibenoberflächen eine Schicht (12, 13) mit lichtabsorbierenden Eigenschaften in der Scheibe anogeordnet ist.

18. Augenvorsatzscheibe nach einem der Ansprüche 1 bis 16, dadurch gekennzeichnet, daß sich wenigstens außerhalb des Hauptsichtfeldes (1) im Abstand voneinander verlaufende Farbbänder (15) ohne oder zumindest geringer Transparenz in der Scheibe erstrecken, die im wesentlichen über die gesamte Dicke der Scheibe reichen.

19. Augenvorsatzscheibe nach Anspruch 16, dadurch gekennzeichnet, daß die Farbbänder (15) ohne oder zumindest geringer Transparenz unter einem von der Senkrechten zur Scheibenoberfläche abweichenden Winkel angeordnet sind.

20 Augenvorsatzscheibe nach Anspruch 18, dadurch gekennzeichnet, daß die Farbbänder (15) ohne oder zumindest geringer Transparenz sich längs der Begrenzungen (4, 5) zwischen den Feldern (1, 2, 3) unterschiedlicher Transmission erstrecken.

21 Augenvorsatzscheibe nach einem der Ansprüche 1 bis 18, dadurch gekennzeichnet, daß die Außenkante der Scheibe von einer im wesentlichen über die Scheibendicke reichenden Schicht (15) ohne oder zumindest geringer Transparenz umschlossen ist.

FIG. 1

FIG. 2

FIG. 3

FIG. 4A    FIG. 4B    FIG. 4C